# FASCICULE DE BREVET EUROPEEN

(11) **EP 2 885 397 B1**
(45) Date de publication et mention de la délivrance du brevet: **30.05.2018**
(21) Numéro de dépôt: 13744668.8
(22) Date de dépôt: 02.07.2013
(51) Int. Cl.: C12N 1/20, A61K 39/00, C12P 19/04, A61K 39/102

(54) **PROCÉDÉ DE PRODUCTION D'ANTIGÈNES HAEMOPHILUS INFLUENZAE TYPE B**
VERFAHREN ZUR HERSTELLUNG VON HAEMOPHILUS INFLUENZAE-TYP-B-ANTIGENEN
METHOD FOR PRODUCING HAEMOPHILUS INFLUENZAE TYPE B ANTIGENS

(30) Priorité: 02.07.2012 FR 1256329
(43) Date de publication de la demande: 24.06.2015
(73) Titulaire: Sanofi Pasteur, 69007 Lyon (FR)
(72) Inventeur: LE HIR, Jérome, F-31000 Toulouse (FR); LOUBIERE, Pascal, F-31450 Donneville (FR); BARBIRATO, Fabien, F-69530 Brignais (FR); LINDLEY, Nicholas, F-81310 Parisot (FR)
(74) Mandataire: Kerneis, Daniéle
(86) Numéro de dépôt international: PCT/FR2013/051549
(87) Numéro de publication internationale: WO 2014/006318

(56) Documents cités:
- WO-A1-00/56365
- WO-A2-2009/007641
- MERRITT J ET AL: "Development and scale-up of a fed-batch process for the production of capsular polysaccharide from Haemophilus influenzae", JOURNAL OF BIOTECHNOLOGY, ELSEVIER SCIENCE PUBLISHERS, AMSTERDAM, NL, vol. 81, no. 2-3, 25 août 2000 (2000-08-25), pages 189-197, XP004210490, ISSN: 0168-1656, DOI: 10.1016/S0168-1656(00)00320-5
- S YERUVA ET AL: "SCREENING OF MEDIUM COMPONENTS FOR POLYRIBOSYL RIBITOL PHOSPHATE PRODUCTION BY HAEMOPHILUS INFLUENZAE TYPE-B USING PLACKETT-BURMAN DESIGN", JOURNAL OF CELL AND TISSUE RESEARCH, vol. 10, no. 3, 2010, pages 2349-2352, XP055061494,
- QU JUN ET AL: "Proteomic expression profiling of Haemophilus influenzae grown in pooled human sputum from adults with chronic obstructive pulmonary disease reveal antioxidant and stress responses", BMC MICROBIOLOGY, BIOMED CENTRAL LTD, GB, vol. 10, no. 1, juin 2010 (2010-06), page 162, XP021073045, ISSN: 1471-2180, DOI: 10.1186/1471-2180-10-162

## Description

La présente invention est relative à un procédé, pour la production à l'échelle industrielle, de polysaccharides capsulaires de *Haemophilus influenzae* type b (Hib), selon lequel une souche de Hib est cultivée dans un milieu de culture particulier, dont la composition est chimiquement définie et qui ne comprend donc aucune source complexe d'azote ou de carbone.
On connait dans l'art antérieur, et notamment dans la demande WO2009007641, des milieux de culture améliorés pour *Haemophilus influenzae* type b, qui ne comprennent pas d'élément de source animale, ainsi que cela est recommandé lorsque la culture de *Haemophilus influenzae* type b a pour but de produire du polysaccharide capsulaire qui sera ensuite introduit comme antigène dans la composition d'un vaccin. Dans les milieux décrits dans cet art antérieur, la source d'azote habituellement constituée par des peptones animales est remplacée par des peptones végétales.
De tels milieux représentent de grands progrès pour la sécurité des produits pharmaceutiques en ce qui concerne notamment l'élimination du risque de transmission de maladies telles que l'encéphalite spongiforme bovine ; ils permettent donc de répondre aux recommandations des autorités de santé.
Cependant, la composition des peptones est susceptible de variations suivant les lots fournis, ce qui peut entrainer des variations dans les quantités de bactéries produites ainsi que dans les rendements en antigènes d'intérêt, ce que les producteurs de vaccins essaient de limiter ou de corriger en modifiant certains paramètres lors de la mise en oeuvre du procédé. Cependant, dans le cadre de la production industrielle d'un produit pharmaceutique, il est souhaitable de pouvoir disposer de procédés robustes nécessitant le moins de modifications possible à chaque mise en oeuvre, et dont les résultats à chaque étape sont reproductibles d'un lot à l'autre.
De tels procédés doivent également permettre des rendements compatibles avec une rentabilité industrielle, le critère n'étant pas, dans le cas de production d'antigènes à partir de bactéries, d'obtenir uniquement la plus grande quantité possible de bactéries, mais également d'obtenir le meilleur rapport entre le titre en antigènes et la quantité de bactéries produites ; il est donc souhaitable que la bactérie soit cultivée dans des conditions orientant son métabolisme vers la production de polysaccharide capsulaire.

A cette fin, la présente invention a pour objet un procédé pour la production à l'échelle industrielle, de polysaccharide capsulaire de *Haemophilus influenzae* type b (PRP) destiné à des fins vaccinales, selon lequel on cultive une souche d'*Haemophilus influenzae* type b (Hib) dans un milieu de culture, on recueille le surnageant de culture que l'on traite pour en extraire le polysaccharide capsulaire, ledit milieu de culture comprenant au moins :
- une source de carbone,
- de la protoporphyrine,
- des sels,
- des acides aminés,
- du NAD ou NADH,
- des vitamines,
- des moyens de régulation du pH,
caractérisé en ce que ledit milieu de culture est chimiquement défini, et comprend au moins du Zinc, en une quantité correspondant à celle comprise entre 2.5 et 80 mg/l de ZnSO₄, 7 H₂O, et en ce que le rapport PRP/LPS (en masse) est supérieur à celui obtenu dans un même milieu ne contenant pas de Zinc.

Grâce à l'invention, on peut à la fois, disposer d'un procédé industriel robuste, et augmenter la production de polyoside capsulaire sans avoir à augmenter la biomasse en conséquence, ce qui permet de réduire la quantité de lipopolysaccharides (LPS) produits par rapport à la quantité de PRP produit.

En outre, l'absence de protéines dans la composition du milieu de culture réduit les besoins nécessaires en produit anti mousse et simplifie l'étape de purification qui permet, à partir du surnageant de culture, d'obtenir l'antigène constitué par le polyoside capsulaire.

Selon l'invention, le milieu de culture comprend des moyens de régulation du pH. Ces moyens de régulation du pH peuvent être constitués par des sels tampon et/ou par des moyens de mesure du pH associés à des moyens d'ajouter au milieu soit un acide, soit une base. Grâce à la régulation du pH, les rendements peuvent être optimisés.

Selon un mode de réalisation, le procédé selon l'invention consiste en outre à conjuguer le PRP produit à une protéine porteuse, telle que la protéine tétanique.

L'invention a également pour objet un procédé de préparation d'une composition vaccinale, selon lequel :
- on prépare, à l'échelle industrielle, un antigène contre *Haemophilus influenzae* type b (Hib), constitué par le polysaccharide capsulaire (PRP), selon le procédé de production de PRP de l'invention,
- on conjugue le polysaccharide capsulaire à une protéine porteuse.

Selon un mode de réalisation, on combine en outre le conjugué obtenu à au moins un ou plusieurs antigènes destinés à la vaccination contre une ou plusieurs des infections suivantes : diphtérie, tetanos, polio, Hépatite B, varicelle, oreillons, rubéole, infections provoquées par *Neisseria meningitidis* ou *Streptococcus pneumoniae,* infections provoquées par le rotavirus, afin d'obtenir une combinaison vaccinale permettant l'immunisation simultanée contre plusieurs maladies.

Selon le procédé de l'invention, le milieu de culture est chimiquement défini, c'est-à-dire que la structure chimique ainsi que la quantité de chacun des composants est connue. Un tel milieu est dépourvu de source complexe d'azote ou de carbone, telles que les peptones, caséines, sérums ou autres et peut donc, si cela est souhaité, être garanti ne contenir aucun élément provenant directement d'un animal.

L'invention est relative à un procédé pour la production, à l'échelle industrielle, de polysaccharide capsulaire de *Haemophilus influenzae* de type b. Un tel procédé permet d'obtenir des rendements en biomasse et en polysaccharides capsulaire ou PRP compatibles avec des contraintes industrielles. Un tel procédé permet notamment d'obtenir, à partir d'une souche de *Haemophilus influenzae* de type b possédant dans son code génétique au moins 2 copies du locus *cap* codant pour les fonctions permettant la synthèse capsulaire, sans régulation particulière du milieu, (telles que pH ou pO₂), une biomasse correspondant à une D.O. à 694 nm d'au moins 2.4, après 12 heures de culture, et une quantité de PRP associée d'au moins 240 mg/litre de milieu de culture. La détermination de la quantité de PRP produite étant mesurée par une technique de chromatographie haute performance d'échanges d'anions couplé à un détecteur ampérométrique pulsé (HPAEC-PAD) (Dionex), ou une méthode donnant des résultats équivalents. Avantageusement, le procédé selon l'invention permet d'obtenir une quantité de PRP d'au moins 270 mg/litre de milieu, et plus avantageusement encore une quantité d'au moins 300 mg/litre.

Parmi les sources possibles pour le carbone, on peut citer toutes celles métabolisables par la souche de *Haemophilus influenzae* type b et notamment: le glucose, le fructose, le galactose, le glycérol, le xylose, le ribose, le fucose, l'acide sialique, le lactate. On peut également utiliser 2 ou plusieurs sources différentes, notamment du lactate et du glucose. Selon l'invention, la quantité de glucose présente dans le milieu au départ est comprise entre 5 et 25g/l, plus particulièrement de 10 à 20g/l, et plus particulièrement de 12 à 16g/l.
La quantité de lactate présente dans le milieu au départ peut être comprise entre 0 et 15g/l, et plus particulièrement de 0.5 à 10g/l.

Selon un mode de réalisation de l'invention, la protoporphyrine du milieu est de la protoporphyrine synthétique par exemple celle fournie par la société Sigma Aldrich sous la référence 258385 (Protoporphyrin disodium salt). Ainsi, il est possible d'utiliser un milieu que l'on peut garantir être complètement dépourvu de substance d'origine animale. Alternativement, on utilise de la Protoporphyrine IX de synthèse, dont la formule est la suivante : 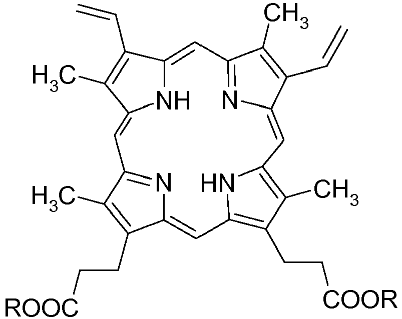 où R désigne H, ou un contre-ion, de préférence un contre-ion de métal alcalin , en particulier le sodium.
Une telle protoporphyrine IX, et sa méthode de préparation, ont été décrites dans la demande de brevet FR2914302.
Selon l'invention, la quantité de protoporphyrine présente dans le milieu au départ est avantageusement comprise entre 0.1 et 10 mg/l, et plus particulièrement entre 0.25 et 2 mg/L.

Le milieu de culture selon le procédé de l'invention comprend des sels qui apportent les minéraux nécessaires à la croissance cellulaire, permettent d'assurer une pression osmotique favorable à la bactérie, et qui exercent en outre un pouvoir tampon sur le pH.
De façon préférée, on utilise un mélange de cations monovalents tels que Na⁺ et/ou K⁺, de cations divalents tels que Ca⁺⁺, Mg⁺⁺, Co⁺⁺, Zn⁺⁺ Mn⁺⁺, Fe⁺⁺, d'anions phosphate sous la forme HPO₄⁻⁻, H₂PO4⁻ et/ou PO4⁻⁻⁻ et d'anions SO₄⁻⁻, et Cl⁻ sous forme de solutions salines dont les molarités de chacune d'entre elles peuvent varier habituellement dans une gamme de concentration allant de 10⁻⁴ mM à 1000 mM.
Les sels présents dans le milieu de culture sont notamment choisis parmi : K₂HPO₄ ; KH₂PO₄ ; MgSO₄, 7 H₂O; Na₂HPO₄, 12 H₂O; NaH₂PO₄, 2 H₂O ; CaCl₂, 2 H₂O; FeSO₄, 7 H₂O ; ZnSO₄, 7 H₂O ; CoCl₂, 6 H₂O; MnSO₄, H₂O.
Les concentrations en sels sont choisies afin d'avoir une osmolarité comprise entre 200 et 700 milliosmole/1, en particulier entre 300 et 400 milliosmole/1, notamment 350 milliosmoles/l, et un pH allant de 6.5 à 7.5.

Pour cela, le milieu de culture selon le procédé de l'invention peut comprendre en particulier :
- MgSO₄, 7 H₂O à une concentration comprise entre 150 et 1500 mg/l,
- CaCl₂, 2 H₂O à une concentration comprise entre 6.5 et 52 mg/l,
- FeSO₄, 7 H₂O à une concentration comprise entre 1.25 et 10 mg/l,
- ZnSO₄, 7 H₂O à une concentration comprise entre 2.5 et 80 mg/l,
- du CoCl₂, 6 H₂O entre 0.5 et 2 mg/l,
- du MnSO₄, H₂O entre 2.5 et 10 mg/l.
- du sodium sous forme notamment de lactate de sodium à 60% en quantité comprise entre 0 et 4 ml/l.
- K₂HPO₄ et KH₂PO₄ à une concentration comprise pour chacun d'entre eux entre 100 et 1200 mg/litre lorsque l'effet tampon est assuré par les Na₂HPO₄, 12 H₂O/NaH₂PO₄, 2 H₂O.
- Na₂HPO₄, 12 H₂O à une concentration comprise entre 15 et 120 g/l,
- NaH₂PO₄, 2 H₂O à une concentration de 10 à 30 fois inférieure à Na₂HPO₄, 12 H₂O.
Alternativement, il est possible que l'effet tampon soit assuré par le couple K₂HPO₄/ KH₂PO₄ ; dans ce cas, la concentration de K₂HPO₄ est comprise entre 15 et 120 g/l, et la concentration de KH₂PO₄ est inférieure de 10 à 30 fois celle de K₂HPO₄ ; la concentration de Na₂HPO₄, 12 H₂O et de NaH₂PO₄, 2 H₂O peut alors être comprise entre 100 et 1200 mg/litre.
Parmi les sels cités, on a pu noter que la présence de Zinc était particulièrement avantageuse pour la production de biomasse et de PRP. En particulier, en concentration allant de 2.5 à 80 mg/l, ou plus particulièrement de 5 à 20 mg/l. De très bons résultats ont été obtenus à une concentration de 20 mg/l.
Dans le cas où le pH du milieu de culture est régulé lors de la phase de culture, par ajout notamment d'une base fortement concentrée telle que NaOH ou KOH, il est possible de réduire la quantité de sels présents dans le milieu, et notamment la quantité des couples tampon Na₂HPO₄/NaH₂PO₄, ou K₂HPO₄/ KH₂PO₄ à condition toutefois de maintenir l'osmolarité à une valeur convenable, c'est-à-dire entre 200 et 700 mOsm/l, par un ajout éventuel de NaCl.

Selon le procédé de l'invention, le milieu de culture comprend également du NAD ou une source de NAD, ou de toute autre coenzyme équivalente. Le NAD (ou β-nicotinamide adenine dinucleotide) encore appelé coenzyme ou facteur V est un facteur de croissance essentiel à la culture de *Haemophilus influenzae* type b. Il est avantageusement présent à une concentration comprise entre 0.5 et 50 mg/l, et plus particulièrement entre 2 et 10 mg/l, en particulier 5 mg/l de milieu de culture. Il peut être apporté au milieu sous sa forme réduite de NADH. Alternativement au NAD, le milieu de culture peut comprendre des éléments précurseurs du NAD que les bactéries seront capables d'utiliser tels que du NADP/NADPH (β-nicotinamide adenine dinucleotide phosphate) ou du NMN (β-nicotinamide adenine mononucleotide) ou du NR (nicotinamide riboside), du 3-Acetylpyridine Adenine Dinucleotide (APAD) ou du 3-Acetylpyridine Mononucleotide (APMN).

Selon le procédé de l'invention le milieu de culture contient des acides aminés qui sont avantageusement choisis parmi : l'arginine, l'alanine, la lysine, l'histidine, le tryptophane, la valine, l'isoleucine, la leucine, la tyrosine, la phenylalanine, la cystine (ou un équivalent), l'asparagine, la glutamine, l'acide aspartique, l'acide glutamique.
Une formulation du milieu de culture comprend de l'arginine, de l'alanine, de l'histidine, du tryptophane, de la tyrosine, de la phenylalanine, de la cystine, de l'acide aspartique et de l'acide glutamique uniquement. Alternativement, l'ensemble des acides aminés cités est présent dans le milieu de culture.
Selon un mode particulier la cystine est remplacée par du glutathion ou de la cystéine.
Selon un mode de réalisation préféré, le procédé selon l'invention met en oeuvre un milieu de culture dépourvu des acides aminés suivants : L- Methionine, L-Glycine, L-Proline, L-Serine, et L-Threonine. En effet, la présence de ces acides aminés a conduit à une augmentation de la biomasse, mais pas à une augmentation correspondante de la production de PRP.

Les acides aminés sont fournis sous forme de poudres très pures fournies par Sigma. De façon particulièrement avantageuse, ces acides aminés ont une origine synthétique ou ont été obtenus grâce à des procédés garantissant le fait qu'ils ne contiennent aucun élément provenant directement d'un animal, afin d'exclure tout risque de contamination par des infections transmissibles, et notamment par l'ESB (Encephalite Spongiforme Bovine). Les quantités de chaque acide aminé sont choisies afin d'optimiser la croissance cellulaire et la production de PRP. Les quantités d'acide aspartique, d'asparagine et de glutamine sont choisies de manière à entrainer une carence lors de la culture cellulaire, ce qui permet d'orienter le métabolisme de la cellule vers la production de polysaccharide capsulaire, alors que les quantités des autres acides aminés sont choisies de manière à ce qu'il n'y ait pas de carence durant la phase de culture.

Selon le procédé de l'invention, le milieu de culture contient en outre des vitamines qui sont choisies parmi : la thiamine, le pantothenate, l'uracile, l'hypoxanthine, la biotine, la riboflavine et la pyridoxine. Au sens de l'invention, le terme de vitamines englobe également l'uracile et l'hypoxanthine qui sont des bases azotées.
De façon particulièrement avantageuse, les vitamines présentes dans le milieu de culture ont une origine non-animale , en particulier une origine synthétique ce qui permet d'exclure tout risque de contamination par des infections transmissibles, et notamment par l'ESB (Encephalite Spongiforme Bovine). On utilise notamment des vitamines fournies par Sigma dont le degré de pureté est connu avec précision, ce qui permet un dosage très précis lors de la préparation du milieu de culture.
Les quantités de vitamines présentes dans le milieu sont choisies afin d'optimiser la production de biomasse et de PRP.

Selon un mode particulier de l'invention, le milieu de culture comprend de la citrulline qui peut se substituer à l'arginine et à l'uracile .
Dans ce cas, la quantité de citrulline comprise dans le milieu de culture est avantageusement comprise entre 150 et 500 mg/l.

Grâce au milieu de culture selon le procédé de l'invention, on a pu produire du Polyribosyl-Ribitol-Phosphate en quantité plus importante que lors de l'utilisation de milieux selon l'art antérieur, tout en ne consommant pas plus de carbohydrate, et en ne produisant pas plus de contaminant, notamment de LPS.

Selon un mode de réalisation de l'invention, la souche de *Haemophilus influenzae* type b mise en culture est une souche capsulée possédant dans son code génétique au moins 2 copies du locus *cap.* Ce locus *cap,* d'une taille comprise entre 17 et 18 kb regroupe des genes dont l'expression est liée à la synthèse et à l'exportation de la capsule. L'expression de la capsule donne une couleur blanche à la bactérie lorsqu'elle est cultivée sur gélose spécifique ; les colonies de bactéries possédant ce locus *cap* sont de ce fait nommées « colonies blanches », alors que les bactéries n'exportant pas le polyoside capsulaire à la surface de la capsule sont appelées « colonies grises ». L'expression de la capsule est soumise à une instabilité génétique potentielle, pouvant entrainer l'apparition de mutants déficiants en capsule, ce qui entrainerait par conséquent une baisse de la production en PRP. Des études ont permis de démontrer que le milieu chimiquement défini mis en oeuvre dans le procédé selon l'invention permettait d'assurer une bonne stabilité génétique de la souche utilisée au cours d'une vingtaine de générations cellulaires.

Selon l'invention, on peut procéder à la culture de la bactérie d'*Haemophilus influenzae* de type b en plusieurs étapes successives de manière à augmenter progressivement la biomasse.
Dans ce cas, les bactéries provenant d'un lyophilisat ou d'un congelât sont ensemencées dans un volume de milieu n'excédant généralement pas 1 litre. Au bout d'une nuit de culture ou lorsque la densité optique du milieu est suffisante, on transfère cette première culture dans un second milieu de culture identique au premier, mais dont le volume peut être jusqu'à 10 à 20 fois plus grand. La quantité de bactéries ensemencées dans le second milieu est ajustée de telle sorte que la densité optique (D.O.) initiale du second milieu de culture à 694 nm soit comprise entre 0,2 et 0,4 pour favoriser une croissance rapide de la population bactérienne. Cette deuxième culture est habituellement réalisée en fermenteur mais d'autres types de récipients peuvent être utilisés (flasks, spinners,...). Lorsque la culture est effectuée en fermenteur, on utilise habituellement pendant la durée de la culture une température de 37 °C +/- 1°C, une agitation constante, une pression de 0,1 bar, une pO₂ de 30% et un débit d'air de 0,25 volume de gaz par volume de milieu par minute. Il est de la compétence de l'homme de l'art de choisir d'autres paramètres pour ce type de culture. A la fin de la phase exponentielle de croissance bactérienne, on peut encore amplifier la biomasse en la transférant dans un autre fermenteur de plus grande capacité en utilisant la même procédure et ainsi de suite. Les volumes de culture obtenus peuvent atteindre, voire dépasser 1000 litres. La(les) culture(s) est (sont) généralement réalisée(s) selon le mode « Batch ».
On prélève finalement le surnageant de la dernière culture après inactivation des bactéries. L'inactivation est classiquement réalisée à l'aide d'une solution de formol à une concentration finale de 0,35%-0,37% (V/V). Le surnageant est classiquement séparé des bactéries par une étape de centrifugation. Le PRP contenu dans le surnageant résultant est ensuite extrait et purifié selon des procédés classiques bien connus de l'homme du métier.
Le PRP récolté et purifié est ensuite avantageusement conjugué à une protéine porteuse telle que la protéine tétanique, afin de le rendre T-dépendant et permettre notamment l'immunisation de jeunes enfants. L'antigène PRP-T ainsi obtenu peut ensuite être utilisé seul dans un vaccin monovalent, ou être combiné à d'autres antigènes afin de permettre la vaccination simultanée contre plusieurs maladies.
De façon particulièrement avantageuse, la présente invention propose un procédé de préparation d'une composition vaccinale selon lequel on combine le conjugué obtenu à au moins un ou plusieurs antigènes présents habituellement dans les vaccins pédiatriques, et notamment des antigènes contre la diphtérie, le tétanos, la poliomyelite, l'Hépatite B, les infections engendrées par *Neisseria meningitidis* ou par *Streptococcus pneumoniae,* la varicelle, les oreillons, la rubéole, les infections provoquées par un rotavirus...
Selon un mode de réalisation, le conjugué PRP-T est présent sous forme de poudre alors que les autres antigènes sont sous forme liquide, l'ensemble des antigènes étant mélangé extemporanément avant administration ; selon un mode de réalisation alternatif, la composition vaccinale est entièrement liquide.
Le procédé de préparation selon l'invention permet ainsi de préparer une combinaison vaccinale quadrivalente comprenant, en plus du conjugué PRP-T, des antigènes de la diphtérie, du tétanos, de l'hépatite B. Alternativement, il permet de préparer une combinaison vaccinale quadrivalente, comprenant, en plus du conjugué PRP-T, de l'anatoxine diphtérique, de l'anatoxine tétanique, les antigènes acellulaires de Bordetella pertussis au nombre de 2 (anatoxine et l'hémagglutinine filamenteuse), ou 3 (les 2 précédents + pertactine) ou encore 5 (les 3 précédents + les aggutinogènes).
Il permet aussi de préparer une combinaison vaccinale pentavalente comprenant, en plus du conjugué PRP-T, de l'anatoxine diphtérique, de l'anatoxine tétanique, les antigènes acellulaires de Bordetella pertussis au nombre de 2, 3 ou 5, ainsi que les virus inactivés de la polio de type 1, 2 et 3.

Il permet aussi de préparer une combinaison vaccinale hexavalente comprenant en plus du conjugué PRP-T, de l'anatoxine diphtérique, de l'anatoxine tétanique, les antigènes acellulaires de Bordetella pertussis au nombre de 2, 3 ou 5, de l'hépatite B, ainsi que les virus inactivés de la polio de type 1, 2 et 3. Une telle combinaison hexavalente peut notamment être liquide et comprendre par dose de 0.5ml :
- l'anatoxine diphtérique en quantité de 20 UI,
- l'anatoxine tétanique en quantité de 40 UI,
- l'antigène de surface de l'hépatite B à raison de 10µg,
- l'anatoxine de Pertussis à raison de 25µg,
- l'hémagglutinine filamenteuse de Pertussis à raison de 25µg,
- du PRP-T à raison de 12µg de PRP,
- les virus polio inactivés de type 1, 2 et 3 en quantité respective de 40, 8 et 32 DU,
- de l'hydroxyde d'aluminium à raison de 0.6 mg d'Al³⁺.

Alternativement, il permet de préparer des combinaisons vaccinales dans lesquelles les antigènes de la coqueluche sont constitués par la bactérie entière *Bordetella pertussis.*

Grâce au procédé de culture selon l'invention, il est possible d'accroitre la production de Polyribosyl Ribitol Phosphate, sans augmenter la quantité de biomasse ce qui permet de réduire la quantité de LPS contaminants ; cet avantage du procédé selon l'invention est très important pour l'industrie du vaccin, où ces produits sont introduits comme antigènes dans des compositions vaccinales, et où les quantités de LPS doivent être réduites au minimum. Le fait de disposer d'un procédé permettant de ne pas augmenter la quantité de LPS produit pour une quantité supérieure de PRP offre la possibilité de simplifier le procédé de purification subséquent.

### Exemple 1: Préparation d'un milieu de culture selon le procédé de l'invention

On a préparé un milieu de culture selon le procédé de l'invention à partir d'un milieu de base auquel on a rajouté extemporanément des solutions d'enrichissement.
La composition du milieu de base est indiquée dans le tableau I ci-après :

**Tableau I :**

| **Composés** | **Quantités en mg pour 1l** | **fournisseur** | **ref. produit** |
|---|---|---|---|
| Lactate de sodium à 60% | 1,5 ml | Prolabo | 27925.292 |
| K₂HPO₄ | 300 | VWR Prolabo | 26931.263 |
| KH₂PO₄ | 300 | VWR Prolabo | 26923.298 |
| MgSO₄ 7H₂0 | 368 | VWR Prolabo | 25165.260 |
| Na₂HPO₄ 12H₂O | 28620 | VWR Prolabo | 28028.298 |
| NaH₂PO₄ 2H₂O | 1870 | VWR Prolabo | 28015.294 |
| L-Arginine | 87 | Sigma | A5006 ou A8094 |
| L-Alanine | 134 | Sigma | A7627 ou A7469 ou A4349 |
| L-Asparagine | 198 | Sigma | A0884 ou A7094 |
| L- Lysine | 140 | Sigma | L5626 ou L8662 ou L7039 |
| L-Glutamine | 220 | Sigma | G3126 ou G8540 ou G5792 |
| L-Histidine | 78 | Sigma | H8000 ou H6034 ou H3911 |
| L-Tryptophane | 200 | Sigma | T0254 ou T8941 ou T4196 |
| L-Valine | 115 | Sigma | V0500 ou V0513 ou V4638 |
| L-Isoleucine | 130 | Sigma | I2752 ou I7403 ou I5281 |
| L-Leucine | 130 | Sigma | L8000 ou L6914 ou L8912 |
| L-Tyrosine | 180 | Sigma | T3754 ou T8566 ou T4321 |
| L-Phenylalanine | 165 | Sigma | P5482 |
| L-Cystine | 61 | Sigma | C8755 ou C7602 ou C5735 |
| L-acide aspartique | 1065 | Sigma | A9256 ou A5474 |
| L-acide glutamique | 1471 | Sigma | G1251 ou G8415 |
| Pyridoxine HCl | 4 | Sigma | P5669 |
| Riboflavine | 0,2 | Sigma | R4500 |
| Thiamine HCl | 4 | Sigma | T4625 |
| Biotine | 4 | Sigma | B4501 |
| Pantothenate de Ca | 4,5 | Sigma | P5710 |
| Uracil | 70 | Sigma | U0750 |
| Hypoxanthine | 20 | Sigma | H9377 |
| FeSO₄ 7H₂O | 2,5 | Adrich | 450278 |
| ZnSO₄ 7H₂O | 5 | Sigma | Z4750 |
| CoCl₂ 6H₂O | 1 | Fulka | 60818 |
| MnSO₄ H₂O | 5 | Sigma | M7634 |
| CaCl₂ 2H₂O | 13 | Panreac | 131232.1211 |

Pour préparer 1 litre de milieu, on a ajouté, dans environ 100 ml d'eau déminéralisée, sous agitation grâce à un barreau aimanté, les différents composés suivant l'ordre décrit dans le tableau ci-dessus. Avant ajout, toutes les poudres avaient été au préalable dissoutes dans un faible volume d'eau déminéralisé avec, dans certains cas, ajout d'acide ou de base.

Ainsi, pour la Valine, l'Isoleucine et la Leucine, il a été nécessaire d'ajouter aux quantités indiquées dans le tableau 140µl de KOH 10N ; à la Tyrosine, il a fallu ajouter 280 µl de KOH 10N ; à la Phenylalanine, il a fallu ajouter 210 µl de HCl à 37%, et à la Cystine, il a fallu ajouter 70µl de HCl à 37%.

Au final, on a ajusté le pH à 7,2 ± 0,1 avec de la KOH 10 N (dans le cas où le pH aurait été trop élevé, on aurait utilisé de l'HCL 37 %), puis on a complété au volume désiré avec de l'eau déminéralisée.
Le milieu a ensuite été stérilisé grâce à une filtration sur filtre ayant un seuil de coupure à 0.22 µm.
Le milieu a ainsi pu être conservé 72 heures à 5 °C.

Avant l'inoculation du milieu, on a ajouté:
- 27.3 ml de glucose à 512.8 g/l préparé à partir de D(+) glucose anhydre fourni par la Société VWR sous la référence 24379.294,
- 5 ml d'une solution de NAD à 1 g/l, fourni par la Société Sigma sous la dénomination β-Nicotinamide Adenine Dinucleotide hydraté (réf. 43410),
- 4 ml d'une solution-mère comprenant de la Protoporphyrine à la concentration de 0.25 g/l et de l'hydroxyde d'ammonium à la concentration de 5 ml/l ; la protoporphyrine étant fournie par la Société Sigma Aldrich sous la dénomination Protoporphyrine IX disodium salt (réf. 258385) et l'hydroxyde d'ammonium par la société VWR sous la dénomination Ammoniac solution 28 % (réf. 21190.292),
afin d'obtenir 1 litre de milieu prêt à être inoculé, et comprenant, outre les éléments décrits dans le Tableau I précédent, les éléments additionnels suivants avec les concentrations telles qu'indiquées dans le tableau II ci-après :

**Tableau II :**

| **Composés** | **Concentration avant inoculation dans le milieu** |
|---|---|
| Protoporphyrine | 1 mg/l |
| Ammoniaque | 17.7 µg/l |
| Glucose anhydre | 14 g/l |
| NAD | 5 mg/l |

### Exemple 2 : Préparation d'un milieu de culture selon l'art antérieur.

On a préparé un milieu de culture selon l'art antérieur en ajoutant à un milieu de base des solutions spécifiques d'enrichissement.
La composition du milieu de base est indiquée dans le tableau III ci-après.

**Tableau III :**

| **Composés** | **Concentration dans le milieu de base** |
|---|---|
| Peptone de pois | 7,42 g/l |
| Lactate de Sodium 60 % | 1,5 ml/l |
| Na₂HPO₄ 12H₂O | 31,14 g/l |
| NaH₂PO₄ 2H₂O | 2,03 g/l |
| Cystine (L) | 0,07 g/l |
| HCl (10N) | 0,07 ml/l |
| Tryptophane (L) | 0,02 g/l |
| (NH₄)₂SO₄ | 1 g/l |
| MgSO₄ 7H₂O | 0,4 g/l |
| CaCl₂ 2H₂O | 0,02 g/l |

Pour préparer ce milieu de base, on a ajouté à 100 ml d'eau déminéralisée maintenue sous agitation grâce à un barreau aimanté, chacun des éléments suivant l'ordre dans lequel ils sont indiqués dans le tableau. Auparavant, chacun des éléments, fourni sous forme de poudre, avait été dissous dans un faible volume d'eau déminéralisée.
La peptone de pois provient de la Société Kerry et est commercialisée sous la référence Hy-pea 7404. Le (NH₄)₂SO₄ est fourni par la Société VWR sous la référence 21333.296. Les autres éléments proviennent des mêmes fournisseurs et sont fournis sous les mêmes références que celles décrites dans l'exemple 1 pour la préparation du milieu selon le procédé de l'invention. Le pH final a été ajusté à 7,2 ± 0,1 avec de la KOH 10N (dans le cas où le pH aurait été trop élevé, on aurait utilisé de l'HCL 37 %).
On a, ensuite, ajouté de l'eau déminéralisée afin d'obtenir le volume désiré, puis on l'a stérilisé par autoclavage, suivant un cycle de 30 minutes à 120 °C.
Une fois stérilisé, le milieu a pu être conservé 15 jours à 4 °C.

A ce milieu de base ont été ajoutées les solutions d'enrichissement A, B, C et D.
La solution A est une solution comprenant du glucose anhydre à la concentration de 512.8 g/l, fournie par la Société VWR sous la dénomination D(+) glucose anhydre (réf. 24379.294).
La solution B est une solution-mère comprenant du NAD à la concentration de 1 g/l, fournie par la Société Sigma sous la dénomination β-Nicotinamide Adenine Dinucléotide hydraté (réf. 43410).
La solution C est une solution-mère comprenant de la Protoporphyrine à la concentration de 0.25 g/l et de l'hydroxyde d'ammonium à la concentration de 5 ml/l ; la protoporphyrine étant fournie par la Société Sigma Aldrich sous la dénomination Protoporphyrine IX disodium salt (réf. 258385) et l'hydroxyde d'ammonium par la société VWR sous la dénomination Ammoniac solution 28 % (réf. 21190.292).
La solution D est une solution concentrée comprenant de l'ultrafiltrat d'extrait autolytique de levure (Ufel) à 125 g/l; elle est fournie par la Société Biospringer sous la dénomination Ultrafiltrat d'extrait autolytique de levure (Ufel) (Réf. springer 0701).

Chacune de ces solutions est stérilisée par filtration sur filtre dont le seuil de coupure est de 0.22µm.
On a ajouté à 1 litre de milieu de base :
- 35.1 ml de solution A
- 5 ml de solution B
- 4 ml de solution C
- 40 ml de solution D

L'ajout de chacune des solutions au milieu de base a été réalisé sous hotte à flux laminaire et a conduit à un milieu de culture ayant avant inoculation, outre les éléments mentionnés dans le tableau III précédent, les éléments additionnels suivants dans la concentration indiquée dans le tableau IV ci-après :

**Tableau IV :**

| **Composés** | **Concentration avant inoculation dans le milieu** |
|---|---|
| Ultrafiltrat d'extrait autolytique de levure (Ufel) | 4,9 g/l |
| Protoporphyrine | 1 mg/l |
| Ammoniaque | 17.7µg/l |
| Glucose anhydre | 18 g/l |
| NAD | 5 mg/l |

### Exemple 3 : Comparaison de la production de PRP avec un milieu selon le procédé de l'invention tel que décrit à l'exemple 1 et un milieu de l'art antérieur tel que décrit à l'exemple 2.

On a réalisé ces expériences comparatives selon un protocole de culture en 2 étapes:
- une étape de préculture en Erlenmeyer en verre de 1 litre sans baffle, à 36°C sous agitation à 130 rpm (rotations par minute), pendant une durée de 8 heures à l'issue de laquelle la souche est en fin de phase linéaire de croissance ; cette préculture est initiée par l'inoculation de 280 ml de milieu de culture par un inoculum de bactéries d'*Haemophilus influenzae* type b contenant au moins 2 copies du locus *cap,* à une concentration de 0.5% (volume/volume).
- puis un transfert de 90 ml de la préculture a permis d'ensemencer un fermenteur Biostat B plus de 2 litres contenant 1.8 litres de milieu de culture. Ce fermenteur a été maintenu à 37° C, sous agitation de 400 rpm avec une aération de 0.45 lpm (litre par minute) pendant 12 heures.
Après 12 heures de culture, on a mesuré la D.O. à 694 nm, ce qui a permis de déterminer la biomasse. En effet, lors d'essais précédents, on a pu déterminer qu'une unité de la densité optique mesurée à 694 nm correspondait à 0.64 g de biomasse (masse sèche)/ litre.
On a déterminé la quantité de PRP total dans le surnageant de culture par chromatographie haute performance d'échange d'anions couplé à un détecteur ampérométrique pulsé (HPAEC-PAD, selon une méthode très proche de celle décrite dans la publication de Sturgess et al. (Vaccine 17 (1999) 1169-1178) . Le surnageant de culture a été filtré par passage sur filtre 0,22 µm puis 500 µl de surnageant a été ultrafiltré via l'utilisation de colonnes Amicon ultra avec un seuil de coupure de 10 KDa (millipore ref. UFC5010BK) et soumis ensuite à une étape d'hydrolyse basique par ajout de NaOH.
L'hydrolyse s'est déroulée à température ambiante durant au minimum 4 heures.
La validité du dosage a été contrôlée par l'utilisation d'un étalon interne, la glucosamine 1 phosphate (GlcN1P), présent dans la solution d'hydrolyse et par le passage régulier d'un contrôle interne ayant une concentration en PRP connue. Par rapport aux indications de Sturgess *et al,* la phase mobile était composée de NaOH 35 mM et de CH₃COONa 114 mM et la phase de régénération a été effectuée durant 10 minutes avec NaOH 100 mM et CH₃COONa 400 mM.
Les résultats obtenus sont indiqués dans le tableau V ci-après :

**Tableau V :**

| | **Milieu selon l'invention (4 essais)** | **Milieu de l'art antérieur (1 essai)** |
|---|---|---|
| D.O. à 694 nm | 2,52 ± 0.14 | 3,9 |
| Biomasse en g de masse sèche/ litre de milieu de culture | 1,74 ± 0.09 | 2,6 |
| Concentration en PRP en mg/ litre de milieu de culture | 349 ± 22 | 169 |
| Production spécifique en mg de PRP/g de biomasse | 199 ± 3 | 66 |

Ces résultats montrent l'intérêt du procédé selon l'invention pour la productivité du PRP : on remarque en effet, que pour un même volume de milieu de culture, si la biomasse obtenue était plus importante avec un milieu de culture selon l'art antérieur, la quantité de PRP était par contre plus faible.

### Exemple 4 : Comparaison de la quantité de PRP et de LPS présente dans un surnageant de culture selon le procédé de l'invention, et dans un surnageant de culture selon un procédé de l'art antérieur.

On a procédé à la mise en culture de *Haemophilus influenzae* type b de façon comparative avec un milieu selon le procédé de l'invention, et avec un milieu selon l'art antérieur, de la manière décrite à l'exemple 3, avec cependant la différence que le milieu de base du procédé selon l'art antérieur comprenait 10 g/l d'hydrolysat acide de caséine fourni par la Société Solabia (réf. A1434) à la place de la peptone de pois présente dans le milieu selon l'exemple 3.
Le temps de pré-culture est, comme à l'exemple 3, de 8 heures, et celui de culture de 12 heures.
Les dosages de PRP et de LPS sont tous deux effectués par HPAEC-PAD ; mais le dosage de LPS est réalisé par quantification d'un monosaccharide particulier, l'heptose, à partir d'une gamme étalon de LPS purifié ayant subi le même protocole, le contrôle interne étant pour ce dosage du rhamnose.
Les résultats obtenus sont indiqués dans le tableau VI ci-après :

**Tableau VI :**

| | **Milieu selon l'invention (4 essais)** | **Milieu selon l'art antérieur (3 essais)** |
|---|---|---|
| DO à 694 nm | 2.5 ± 0.1 | 3.9 ± 0.3 |
| Biomasse en g de masse sèche/l de milieu | 1.7 ± 0.1 | 2.5 ± 0.2 |
| Concentration en LPS en mg/l de milieu | 5 ± 1 | 4.6 ± 1 |
| Concentration en PRP en mg/l de milieu | 349 ± 22 | 17 ± 5 |
| Rapport PRP/LPS | 74 ± 15 | 38 ± 6 |

Les résultats obtenus montrent l'intérêt du procédé selon l'invention, qui permet de réduire la quantité de LPS produits par rapport à la quantité de PRP.
Ceci représente un avantage important du procédé selon l'invention, car cela simplifie beaucoup les opérations de purification, tout en maintenant un niveau de sécurité approprié.

### Exemple 5 : Essai permettant d'évaluer l'importance du Zinc.

On a étudié l'importance de la présence de Zinc dans le milieu de culture.

A cet effet, on a comparé 3 milieux :
- un milieu tel que celui décrit à l'exemple 1 appelé MS,
- un milieu ayant une composition identique à celui de l'exemple 1 mais sans le ZnSO₄, appelé MS Zn-,
- un milieu tel que celui décrit à l'exemple 1 mais avec une concentration en ZnSO₄ 4 fois supérieure, soit 20 mg/l et appelé MS Zn++.
Les milieux ont ensuite été stérilisés grâce à une filtration sur un filtre ayant un seuil de coupure à 0.22 µm.
On a tout d'abord procédé à une étape de préculture en Erlenmeyer en verre de 1 litre, sans baffle, pendant 16 heures à 36°C sous agitation à 130 rpm, en ensemençant 280 ml d'un milieu tel que décrit à l'exemple 4 par un congelât de bactéries *Haemophilus influenzae* type b possédant dans son code génétique au moins 2 copies du locus *cap.* Puis 9,5 ml de pré-culture ont été lavés dans chacun des milieux de culture à tester avant l'ensemencement pour la culture qui s'est déroulée à chaque fois dans un Erlenmeyer bafflé de 500 ml en polycarbonate contenant 170 ml du milieu de culture à tester ; la culture a été effectuée pendant 12 heures à 37 °C et une agitation à 150 rpm.
On a, pour chaque milieu, déterminé la biomasse, la concentration en PRP ainsi que la quantité de LPS en fin de culture, de la même manière que décrit aux exemples 3 et 4, et calculé les rapports PRP/biomasse ainsi que LPS/biomasse et PRP/LPS.
Les résultats obtenus sont indiqués dans le tableau VII ci-après :

**Tableau VII :**

| | **MS** | **MS Zn-** | **MS Zn++** |
|---|---|---|---|
| Biomasse en g de masse sèche/litre de milieu de culture | 1.98 | 1.23 | 2.22 |
| Concentration en PRP en mg/litre de milieu de culture | 319 | 203 | 372 |
| Production spécifique en mg de PRP/g de biomasse | 160 | 165 | 167 |
| Concentration en LPS en mg/litre de milieu de culture | 11.5 | 8.8 | 12.1 |
| Rapport de LPS/biomasse en mg/g de biomasse | 6.1 | 7.2 | 5.5 |
| Rapport de PRP/LPS en mg/mg | 30 | 23 | 31 |

Ces résultats ont montré que la présence de zinc dans le milieu de culture permet d'augmenter significativement la production de biomasse ainsi que la production de PRP, sans pour autant accroître la quantité de LPS proportionnellement.

### Exemple 6 : Essais en fermenteur de 5 litres.

On a procédé à la mise en culture de *Haemophilus influenzae* type b dans le milieu de culture décrit selon l'invention à l'exemple 1 en utilisant uniquement des produits garantis par les fournisseurs comme étant animal-free et en utilisant de la protoporphyrine de synthèse telle que décrite dans la demande de brevet FR2914302, et fournie par la société SOLVIAS AG sous la désignation : Protoporphyrin IX Disodium Salt (Ref. SOL20402). Les essais se sont ensuite déroulés de la manière décrite dans l'exemple 3 à l'exception du fait que les fermenteurs utilisés étaient des fermenteurs 5 litres régulés à 37 °C, 550 rpm d'agitation et 0.5 lpm d'aération. Le volume d'inoculum était alors de 280 ml soit la totalité de l'erlenmeyer de préculture.

La détermination de la concentration en PRP et LPS a été faite suivant les procotoles décrits aux exemples 3 et 4.

Les résultats obtenus sont indiqués dans le Tableau VIII ci-après :

**Tableau VIII :**

| | **Milieu animal free selon l'invention** |
|---|---|
| D.O.à 694 nm | 3.58 |
| Biomasse en g de masse sèche/l de milieu | 2.29 |
| Concentration en PRP en mg/l de milieu | 567 |
| Production spécifique en mg de PRP/g de biomasse | 247 |

Ces résultats confirment les résultats précédents, et montrent l'intérêt du procédé selon l'invention pour l'obtention d'une grande quantité de PPR.

### Exemple 7 : Comparaison de la production de biomasse et de PRP avec un milieu selon l'invention tel que décrit à l'exemple 1 et avec des milieux chimiquement définis décrits dans l'art antérieur.

On a procédé à la mise en culture de *Haemophilus influenzae* type b dans le milieu de culture décrit selon l'invention à l'exemple 1, ainsi que dans 8 milieux décrits dans des publications en liaison avec la culture de *Haemophilus influenzae,* et présentés comme étant chimiquement définis.
Ces 8 milieux ont été préparés suivant les recommandations des auteurs. Il s'agit chronologiquement des milieux :
- de Talmadge et Herriott : Biochemical and Biophysical Research Communications, (March 1960), Vol.2 N°3, p 203-206),
- Butler : J. gen. Microbiol. (1962), 27, 51-60,
- Wolin : J. Bacteriol. Vol.85, (1963) Notes, p 253-254,
- Herriott et al. : Journal of Bacteriology, (Feb. 1970), Vol. 101, N°2, 513-516,
- Klein et Luginbuhl : Journal of General Microbiology (1979), 113, 409-411
- Coleman et al. : Journal of Clinical Microbiology, (Sept 2003), Vol.41, N°9 p. 4408-4410.
Les références des produits utilisés pour la préparation de ces 8 milieux sont reproduites dans le tableau IX ci-après :

**Tableau IX :**

| **Produits** | **Fournisseur s** | **ref. produit** |
|---|---|---|
| Uridine | Sigma | U3003 |
| Inosine | Sigma | I4125 |
| Adenosine | Sigma | A4036 |
| Guanine | Sigma | G6779 |
| Guanosine | Sigma | G6264 |
| Vitamine B12 | Sigma | V2876 |
| Citrulline | Sigma | C7629 |
| Choline chloride | Fulka | 26978 |
| Thimidine | Sigma | T9250 |
| Inositol | Sigma | I5125 |
| Putrescine dihydrochloride | Sigma | P5780 |
| Nicotinamide | Sigma | N0636 |
| Hematin porcine | Sigma | H3281 |
| Hemin | Fulka | 51280 |
| Sodium oléate | Sigma | O7501 |
| Sodium acétate | Fulka | 71185 |
| Folique acid | Sigma | F7876 |
| Polyinyl alcohol | Sigma | 341584 |
| Triethanolamine | Sigma | 90278 |
| Glycylglycine buffer | libios | B-GLYGLY250 |
| Tween 40 | Sigma | P1504 |
| Tween 80 | Sigma | P1754 |
| Tris buffer | VWR | 33621.260 |
| éthylènediaminetétraacétate | VWR | 20302.180 |
| HEPES | Sigma | H3375 |
| Glutathion | Sigma | G6013 |
| Adénine | Sigma | A8626 |
| Proline | Sigma | P0380 |
| Thréonine | Sigma | T8625 |
| Glycine | Sigma | G7126 |
| Méthionine | Sigma | M9625 |
| Sérine | Sigma | S4500 |
| Glycerol | VWR | 24387.292 |
| K₂SO₄ | VWR | 26994.293 |
| KCl | VWR | 26764.298 |
| MgCl₂ 6H₂O | Panreac | 131396.1211 |
| NaCl | VWR | 27810.295 |
| NaHCO₃ | VWR | 27778.260 |
| NH₄Cl | VWR | 21236.291 |
| RPMI 1640 avec L-glutamine et 25 mM HEPES | Invitrogen | anciene ref. citée dans la publication de Coleman (2003) ref. 61870036 => nouvelle ref. 52400025 |
| Sodium pyruvate MEM 100 mM | Invitrogen | anciene ref. citée dans la publication de Coleman (2003) ref. 11360070 => nouvelle ref. 11360039 |

Les milieux ont ensuite été stérilisés grâce à une filtration sur un filtre ayant un seuil de coupure à 0.22 µm.
On a tout d'abord procédé à une étape de préculture en Erlenmeyer en verre de 1 litre, sans baffle, pendant 16 heures à 36°C sous agitation à 130 rpm, en ensemençant 280 ml d'un milieu tel que décrit à l'exemple 4 par un congelât de bactéries *Haemophilus influenzae* type b possédant dans son code génétique au moins 2 copies du locus *cap.* Puis 9,5 ml de pré-culture ont été lavés dans chacun des milieux de culture à tester avant l'ensemencement pour la culture qui s'est déroulée à chaque fois dans un Erlenmeyer bafflé de 500 ml en polycarbonate contenant 170 ml du milieu de culture à tester ; la culture a été effectuée pendant 12 heures à 37 °C et 150 rpm d'agitation.
Chaque essai a été réalisé au moins 3 fois.

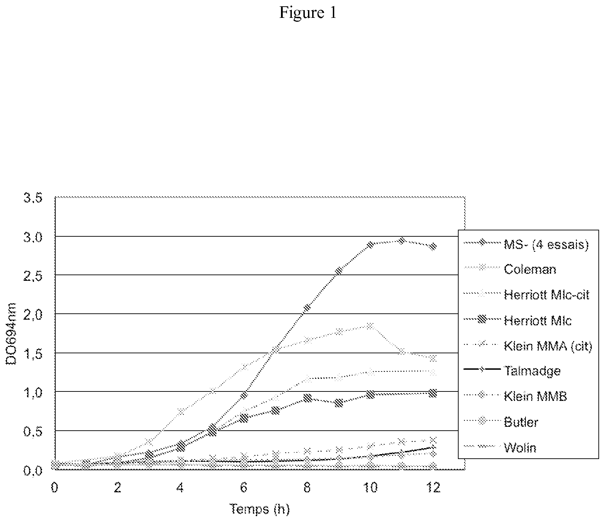

On a, pour chaque milieu, chaque heure, mesuré la D.O. à 694 nm et déterminé la concentration en PRP en fin de culture, de la même manière que décrit à l'exemple 3. Les résultats de D.O. sont reproduits sur la Figure 1.
Les résultats des détermination de PRP sont indiqués dans le tableau X ci-après :

**Tableau X :**

| à 12h | **D.O. à 694nm** | **PRP (mg/l)** |
|---|---|---|
| Milieu selon l'invention | 2.86 ± 0,12 | 320 ± 15 |
| Talmadge | 0.28 ± 0,10 | 20 ± 1 |
| Butler | 0.04 ± 0,00 | 2 ± 0 |
| Wolin | 0.04 ± 0,00 | 6 ± 0 |
| Herriot Mic-cit | 1.26 ± 0,15 | 138 ± 7 |
| Herriot Mic | 0.98 ± 0,13 | 101 ± 5 |
| Klein MMA (cit) | 0.38 ± 0,01 | 40 ± 2 |
| Klein MMB | 0.20 ± 0,03 | 27 ± 1 |
| Coleman | 1.43 ± 0,04 | 204 ± 10 |

### Exemple 8 : Production de PRP à l'échelle industrielle (1000 litres)

On a préparé un milieu de culture selon l'invention ayant la composition de l'exemple 1 exepté pour le zinc où la concentration est de 20 mg/L au lieu de 5 mg/L. En effet l'exemple 5 a montré que cette concentration permettait de produire plus de PRP sans accroitre la quantité de LPS.
Le milieu était identique pour les précultures et la culture finale, excepté le pH (pH initial à 7,2 ± 0,1 puis pH libre pour les précultures et pH régulé à 6,7 ± 0,1 pour la culture de production).

Le milieu a été préparé comme dans l'exemple 1. Après avoir mélangé les composés préalablement dissous dans un faible volume d'eau purifiée, avec dans certains cas ajout d'acide ou de base (cf exemple 1), et avant de faire le QSP avec l'eau purifiée, le pH a été ajusté à 7,2 ± 0,1 pour les précultures et 6,7 ± 0,1 pour la culture finale (avec de la potasse ou soude 10 N ou de l'HCl 37 %).
Le milieu a ensuite été stérilisé grâce à une filtration sur filtre ayant un seuil de coupure à 0.22 µm.
Le milieu a ainsi pu être conservé 72 heures à 5 °C.
Avant l'inoculation du milieu, on a ajouté:
- du glucose à 512.8 g/l préparé à partir de D(+) glucose anhydre fourni par la Société VWR sous la référence 24379.294,
- une solution de NAD à 1 g/l, fourni par la Société Sigma sous la dénomination β-Nicotinamide Adenine Dinucleotide hydraté (réf. 43410),
- une solution-mère comprenant de la Protoporphyrine à la concentration de 0.25 g/l et de l'hydroxyde d'ammonium à la concentration de 5 ml/l; la protoporphyrine étant fournie par la Société SOLVIAS AG sous la dénomination Protoporphyrin IX disodium salt (réf. SOL20402) et l'hydroxyde d'ammonium par la société VWR sous la dénomination Ammoniac solution 28 % (réf. 21190.292),
afin d'obtenir un milieu prêt à être inoculé, et comprenant, outre les éléments décrits dans le Tableau I de l'exemple 1, les éléments additionnels avec les concentrations telles qu'indiquées dans le tableau II de l'exemple 1 (excepté le glucose à une concentration finale de 14,87 g/L au lieu de 14 g/L).

Le procédé à l'échelle 1000 litres comprenait une série de 3 précultures :
- les premières précultures ont été réalisées en fiole Erlenmeyer de 1 litre, sans baffle, contenant 290 mL de milieu de culture complet, à 37°C sous agitation à 130 rpm (rotations par minute), pendant une durée de 17-18 heures. Ces précultures ont été initiées par l'inoculation du milieu de culture par un inoculum de bactéries d'*Haemophilus influenzae* type b contenant au moins 2 copies du locus *cap,* avec un taux d'inoculation correspondant à une DO_{694 nm} cible initiale de 0,014.
- la deuxième série de précultures a été réalisée en fermenteur de 6,8 litres. Deux fermenteurs contenant 5,2 litres de milieu de culture complet ont été ensemencés chacun avec 260 ml d'une préculture de la série 1. Ces fermenteurs ont été maintenus pendant 4 heures à 37° C ± 1, avec un pH initial à 7,2 ± 0,2, une pO2 maintenue à 30% avec une cascade impliquant une augmentation de l'agitation (500 à 800 rpm) puis une augmentation de l'aération (0,5 à 2,5 lpm) et ensuite un débit d'O2 pur entre 0 et 6 lpm.
- la troisième série de préculture a été réalisée dans un fermenteur de 120 litres contenant 57 litres de milieu complet qui a été ensemencé avec 5,8 litres de la préculture provenant de la série 2. Ce fermenteur a été maintenu pendant 3 heures 10 à 37° C ± 1, avec un pH initial de 7,2 ± 0,2, une pO2 maintenue à 30% avec une cascade impliquant une augmentation de l'agitation (300 à 425 rpm) puis une augmentation de l'aération (6 à 28 lpm) et ensuite un débit d'O2 pur entre 0 et 50 lpm.

La culture industrielle a été réalisée dans un fermenteur de 1000 litres contenant 778 litres de milieu complet qui a été ensemencé avec 39 litres d'une préculture de la série 3. Ce fermenteur a été maintenu à 32° C ± 1, avec un pH régulé à 6,7 ± 0,2 (avec une solution de soude à 2,5N), une pO2 maintenue à 70% grâce à une cascade impliquant une augmentation de l'agitation (100 à 230 rpm) puis une augmentation de l'aération (70 à 150 lpm) et ensuite un débit d'O2 pur entre 0 et 500 lpm. Par ailleurs de l'antimousse (Biospumex à 4%) a été ajouté à la demande en fonction du niveau de la mousse.

Après 12 heures de culture, on a mesuré la D.O. à 694 nm, ce qui a permis de déterminer la biomasse (selon la correspondance une unité de DO correspondant à 0,64 g de biomasse sèche). La détermination de la concentration en PRP et LPS a été faite suivant les procotoles décrits aux exemples 3 et 4.
Les résultats obtenus sont indiqués dans le tableau XI ci-après :

**Tableau XI :**

| | **Milieu selon l'invention (1 essai)** |
|---|---|
| D.O. à 694 nm | 3,45 |
| Biomasse en g de masse sèche/ litre de milieu de culture | 2,21 |
| Concentration en PRP en mg/ litre de milieu de culture | 865 |
| Production spécifique en mg de PRP/g de biomasse | 392 |
| Concentration en LPS en mg/litre de milieu de culture | 35,6 |
| Rapport de LPS/biomasse en mg/g de biomasse | 16,1 |
| Rapport de PRP/LPS en mg/mg | 24,3 |

Ces résultats ont montré l'intérêt du procédé selon l'invention pour la productivité du PRP et le ration PRP/LPS, résultats démontrés à l'échelle industrielle.

## Revendications

1. Procédé pour la production à l'échelle industrielle, de polysaccharide capsulaire de *Haemophilus influenzae* type b (PRP) destiné à des fins vaccinales, selon lequel on cultive une souche d'*Haemophilus influenzae* type b (Hib) dans un milieu de culture, on recueille le surnageant de culture que l'on traite pour en extraire le polysaccharide capsulaire, ledit milieu de culture comprenant au moins :
- une source de carbone,
- de la protoporphyrine,
- des sels,
- des acides aminés,
- du NAD ou NADH,
- des vitamines,
- des moyens de régulation du pH,
**caractérisé en ce que** ledit milieu de culture est chimiquement défini, et comprend au moins du Zinc, en une quantité correspondant à celle comprise entre 2.5 et 80 mg/l de ZnSO₄, 7 H₂O , et **en ce que** le rapport PRP/LPS en masse est supérieur à celui obtenu dans un même milieu ne contenant pas de Zinc.

2. Procédé selon la revendication précédente, **caractérisé en ce que** lesdits moyens de régulation du pH sont constitués par des sels tampon.

3. Procédé selon une des revendications précédentes, **caractérisé en ce que** ladite source de carbone peut être multiple et est choisie parmi : glucose, fructose, galactose, glycérol, xylose, ribose, fucose, acide sialique, lactate.

4. Procédé selon une des revendications précédentes, **caractérisé en ce que** ladite protoporphyrine est de la protoporphyrine IX de synthèse.

5. Procédé selon une des revendications précédentes, **caractérisé en ce que** lesdits sels sont choisis parmi les sels de potassium, de magnésium, de sodium, de calcium, de fer, de zinc, de cobalt et de manganèse.

6. Procédé selon la revendication précédente, **caractérisé en ce que** lesdits sels sont choisis parmi: K₂HPO₄ ; KH₂PO₄ ; MgSO₄, 7 H₂O; Na₂HPO₄, 12 H₂O; NaH₂PO₄, 2 H₂O ; CaCl₂, 2 H₂O; FeSO₄, 7 H₂O ; ZnSO₄, 7 H₂O ; CoCl₂, 6 H₂O; MnSO₄, H₂O.

7. Procédé selon une des revendications précédentes, **caractérisé en ce que** lesdits acides aminés sont choisis parmi :
• l'arginine,
• l'alanine,
• un au moins parmi : l'asparagine, la glutamine, l'acide aspartique, l'acide glutamique.
• la lysine,
• l'histidine,
• le tryptophane,
• la valine,
• l'isoleucine,
• la leucine,
• la tyrosine,
• la phenylalanine,
• la cystine ou équivalent.

8. Procédé selon la revendication précédente, **caractérisé en ce que** ledit milieu de culture comprend au moins de l'arginine, de l'alanine, de l'histidine, du tryptophane, de la tyrosine, de la phenylalanine, de la cystine, de l'acide aspartique et de l'acide glutamique.

9. Procédé selon une des revendications précédentes, **caractérisé en ce que** la cystine est remplacé par du glutathion ou de la cystéine.

10. Procédé selon une des revendications précédentes, **caractérisé en ce que** lesdites vitamines sont choisies parmi : la thiamine, le pantothenate, l'uracile, l'hypoxanthine, la biotine, la riboflavine et la pyridoxine.

11. Procédé selon une des revendications précédentes, **caractérisé en ce que** l'arginine et l'uracile sont remplacées par de la citrulline.

12. Procédé de préparation d'une composition vaccinale, selon lequel :
- a) on prépare, à l'échelle industrielle, un antigène contre *Haemophilus influenzae* type b (Hib), constitué par le polysaccharide capsulaire (PRP), selon le procédé de la revendication 1,
- b) on conjugue le polysaccharide capsulaire obtenu à l'étape a) à une protéine porteuse.

13. Procédé selon la revendication précédente, **caractérisé en ce que** la protéine porteuse est l'anatoxine tétanique.

14. Procédé selon une des revendications 12 ou 13, **caractérisé en ce qu'**on combine le conjugué obtenu à l'étape b) à au moins un ou plusieurs antigènes présents habituellement dans les vaccins pédiatriques.

15. Procédé de préparation d'une composition vaccinale selon la revendication 12 ou 13, **caractérisé en ce qu'**on combine le conjugué obtenu à l'étape b) avec des antigènes de la diphtérie, du tétanos, et de l'hépatite B.

16. Procédé de préparation d'une composition vaccinale selon la revendication 12 ou 13, **caractérisé en ce qu'**on combine le conjugué obtenu à l'étape b) avec de l'anatoxine diphtérique, de l'anatoxine tétanique, les antigènes acellulaires de *Bordetella pertussis* au nombre de 2 que sont l'anatoxine et l'hémagglutinine filamenteuse.

17. Procédé de préparation d'une composition vaccinale selon la revendication 12 ou 13, **caractérisé en ce qu'**on combine le conjugué obtenu à l'étape b) avec de l'anatoxine diphtérique, de l'anatoxine tétanique, les antigènes acellulaires de *Bordetella pertussis* au nombre de 3 que sont l'anatoxine, la pertactine et l' hémagglutinine filamenteuse.

18. Procédé de préparation d'une composition vaccinale selon la revendication 12 ou 13, **caractérisé en ce qu'**on combine le conjugué obtenu à l'étape b) avec de l'anatoxine diphtérique, de l'anatoxine tétanique, les antigènes acellulaires de *Bordetella pertussis* au nombre de 5 que sont l'anatoxine, la pertactine, les agglutinogènes et l'hémagglutinine filamenteuse.

19. Procédé de préparation d'une composition vaccinale selon la revendication 12 ou 13, **caractérisé en ce qu'**on combine le conjugué obtenu à l'étape b) avec de l'anatoxine diphtérique, de l'anatoxine tétanique, les antigènes acellulaires de *Bordetella pertussis* au nombre de 2, 3 ou 5 ainsi que les virus inactivés de la polio de type 1, 2 et 3.

20. Procédé de préparation d'une composition vaccinale selon la revendication 12 ou 13, **caractérisé en ce qu'**on combine le conjugué obtenu à l'étape b) avec de l'anatoxine diphtérique, de l'anatoxine tétanique, des antigènes de l'hépatite B, les antigènes acellulaires de *Bordetella pertussis* au nombre de 2, 3 ou 5 ainsi que les virus inactivés de la polio de type 1, 2 et 3.

21. Procédé de préparation d'une composition vaccinale selon la revendication 12 ou 13, **caractérisé en ce qu'**on combine le conjugué obtenu à l'étape b) avec la bactérie entière de *Bordetella pertussis.*

22. Procédé de préparation d'une composition vaccinale selon la revendication 12 ou 13, **caractérisé en ce qu'**on combine le conjugué obtenu à l'étape b) avec l'un au moins des antigènes contre la diphtérie, le tétanos, la poliomyélite, l'hépatite B, les infections engendrées par *Neisseria meningitidis* ou par *Streptococcus pneumoniae.*

## Patentansprüche

1. Verfahren zur Herstellung von Kapselpolysaccharid vom *Haemophilus influenzae* Typ b (PRP) im industriellen Maßstab zu Impfzwecken, bei dem man einen Stamm von *Haemophilus influenzae* Typ b (Hib) in einem Kulturmedium kultiviert, den Kulturüberstand erntet und behandelt, um das Kapselpolysaccharid daraus zu extrahieren, wobei das Kulturmedium mindestens Folgendes umfasst:
- eine Kohlenstoffquelle,
- Protoporphyrin,
- Salze,
- Aminosäuren,
- NAD oder NADH,
- Vitamine,
- Mittel zur pH-Regulation,
**dadurch gekennzeichnet, dass** das Kulturmedium chemisch definiert ist und mindestens Zink in einer Menge, die derjenigen von zwischen 2,5 und 80 mg/l ZnSO₄, 7 H₂O entspricht, umfasst, und dadurch, dass das Verhältnis PRP/LPS, bezogen auf die Masse, größer als dasjenige ist, das in dem gleichen Medium, das kein Zink enthält, erhalten wird.

2. Verfahren nach dem vorhergehenden Anspruch, **dadurch gekennzeichnet, dass** die Mittel zur pH-Regulation aus Puffersalzen bestehen.

3. Verfahren nach einem der vorhergehenden Ansprüche, **dadurch gekennzeichnet, dass** die Kohlenstoffquelle mehrfach sein kann und ausgewählt ist aus: Glucose, Fructose, Galactose, Glycerin, Xylose, Ribose, Fucose, Sialinsäure, Lactat.

4. Verfahren nach einem der vorhergehenden Ansprüche, **dadurch gekennzeichnet, dass** das Protoporphyrin synthetisches Protoporphyrin IX ist.

5. Verfahren nach einem der vorhergehenden Ansprüche, **dadurch gekennzeichnet, dass** die Salze aus Kalium-, Magnesium-, Natrium-, Calcium-, Eisen-, Zink-, Kobalt- und Mangansalzen ausgewählt sind.

6. Verfahren nach dem vorhergehenden Anspruch, **dadurch gekennzeichnet, dass** die Salze aus den folgenden ausgewählt sind: K₂HPO₄; KH₂PO₄; MgSO₄, 7 H₂O; Na₂HPO₄, 12 H₂O; NaH₂PO₄, 2 H₂O; CaCl₂, 2 H₂O; FeSO₄, 7 H₂O; ZnSO₄, 7 H₂O; CoCl₂, 6 H₂O und MnSO₄, H₂O.

7. Verfahren nach einem der vorhergehenden Ansprüche, **dadurch gekennzeichnet, dass** die Aminosäuren aus den folgenden ausgewählt sind:
• Arginin,
• Alanin,
• mindestens einem aus: Asparagin, Glutamin, Asparaginsäure, Glutaminsäure,
• Lysin,
• Histidin,
• Tryptophan,
• Valin,
• Isoleucin,
• Leucin,
• Tyrosin,
• Phenylalanin,
• Cystin oder dergleichen.

8. Verfahren nach dem vorhergehenden Anspruch, **dadurch gekennzeichnet, dass** das Kulturmedium mindestens Arginin, Alanin, Histidin, Tryptophan, Tyrosin, Phenylalanin, Cystin, Asparaginsäure und Glutaminsäure umfasst.

9. Verfahren nach einem der vorhergehenden Ansprüche, **dadurch gekennzeichnet, dass** das Cystin durch Glutathion oder Cystein ersetzt ist.

10. Verfahren nach einem der vorhergehenden Ansprüche, **dadurch gekennzeichnet, dass** die Vitamine aus den folgenden ausgewählt sind: Thiamin, Pantothenat, Uracil, Hypoxanthin, Biotin, Riboflavin und Pyridoxin.

11. Verfahren nach einem der vorhergehenden Ansprüche, **dadurch gekennzeichnet, dass** Arginin und Uracil durch Citrullin ersetzt sind.

12. Verfahren zur Herstellung einer Impfstoffzusammensetzung, bei dem man:
- a) im industriellen Maßstab ein Antigen gegen *Haemophilus influenzae* Typ b (Hib), das aus dem Kapselpolysaccharid (PRP) besteht, gemäß dem Verfahren nach Anspruch 1 herstellt,
- b) das in Schritt a) erhaltene Kapselpolysaccharid an ein Trägerprotein konjugiert.

13. Verfahren nach dem vorhergehenden Anspruch, **dadurch gekennzeichnet, dass** es sich bei dem Trägerprotein um das Tetanus-Toxoid handelt.

14. Verfahren nach einem der Ansprüche 12 oder 13, **dadurch gekennzeichnet, dass** man das in Schritt b) erhaltene Konjugat mit mindestens einem oder mehreren Antigen(en) kombiniert, das/die üblicherweise in pädiatrischen Impfstoffen enthalten ist/sind.

15. Verfahren zur Herstellung einer Impfstoffzusammensetzung nach Anspruch 12 oder 13, **dadurch gekennzeichnet, dass** man das in Schritt b) erhaltene Konjugat mit den Diphtherie-, Tetanus- und Hepatitis-B-Antigenen kombiniert.

16. Verfahren zur Herstellung einer Impfstoffzusammensetzung nach Anspruch 12 oder 13, **dadurch gekennzeichnet, dass** man das in Schritt b) erhaltene Konjugat mit dem Diphtherie-Toxoid, dem Tetanus-Toxoid, zwei azellulären *Bordetella-pertussis-*Antigenen, nämlich dem Toxoid und dem filamentösen Hämagglutinin, kombiniert.

17. Verfahren zur Herstellung einer Impfstoffzusammensetzung nach Anspruch 12 oder 13, **dadurch gekennzeichnet, dass** man das in Schritt b) erhaltene Konjugat mit dem Diphtherie-Toxoid, dem Tetanus-Toxoid, drei azellulären *Bordetella-pertussis-*Antigenen, nämlich dem Toxoid, dem Pertactin und dem filamentösen Hämagglutinin, kombiniert.

18. Verfahren zur Herstellung einer Impfstoffzusammensetzung nach Anspruch 12 oder 13, **dadurch gekennzeichnet, dass** man das in Schritt b) erhaltene Konjugat mit dem Diphtherie-Toxoid, dem Tetanus-Toxoid, fünf azellulären *Bordetella-pertussis-*Antigenen, nämlich dem Toxoid, dem Pertactin, den Agglutinogenen und dem filamentösen Hämagglutinin, kombiniert.

19. Verfahren zur Herstellung einer Impfstoffzusammensetzung nach Anspruch 12 oder 13, **dadurch gekennzeichnet, dass** man das in Schritt b) erhaltene Konjugat mit dem Diphtherie-Toxoid, dem Tetanus-Toxoid, zwei, drei oder fünf azellulären *Bordetella-pertussis-*Antigenen sowie inaktivierten Polioviren des Typs 1, 2 oder 3 kombiniert.

20. Verfahren zur Herstellung einer Impfstoffzusammensetzung nach Anspruch 12 oder 13, **dadurch gekennzeichnet, dass** man das in Schritt b) erhaltene Konjugat mit dem Diphtherie-Toxoid, dem Tetanus-Toxoid, Hepatitis-B-Antigenen, zwei, drei oder fünf azellulären *Bordetella-pertussis*-Antigenen sowie inaktivierten Polioviren des Typs 1, 2 oder 3 kombiniert.

21. Verfahren zur Herstellung einer Impfstoffzusammensetzung nach Anspruch 12 oder 13, **dadurch gekennzeichnet, dass** man das in Schritt b) erhaltene Konjugat mit dem gesamten *Bordetella-pertussis*-Bakterium kombiniert.

22. Verfahren zur Herstellung einer Impfstoffzusammensetzung nach Anspruch 12 oder 13, **dadurch gekennzeichnet, dass** man das in Schritt b) erhaltene Konjugat mit mindestens einem der Antigene gegen Diphtherie, Tetanus, Poliomyelitis, Hepatitis B, von durch *Neisseria meningitidis* oder *Streptococcus pneumoniae* hervorgerufene Infektionen kombiniert.

## Claims

1. Process for producing, on an industrial scale, capsular polysaccharide of *Haemophilus influenzae* type b (PRP) intended for vaccine purposes, according to which a strain of *Haemophilus influenzae* type b (Hib) is cultured in a culture medium, and the culture supernatant is harvested and treated in order to extract the capsular polysaccharide therefrom, said culture medium comprising at least:
- one source of carbon,
- protoporphyrin,
- salts,
- amino acids,
- NAD or NADH,
- vitamins,
- pH-regulating means,
**characterized in that** said culture medium is chemically defined, and comprises at least zinc, in an amount corresponding to the amount between 2.5 and 80 mg/l of ZnSO₄. 7 H₂O, and **in that** the PRP/LPS weight ratio is greater than that obtained in the same medium containing no zinc.

2. Process according to the preceding claim, **characterized in that** said pH-regulating means consist of buffer salts.

3. Process according to either of the preceding claims, **characterized in that** said source of carbon may be multiple and is chosen from: glucose, fructose, galactose, glycerol, xylose, ribose, fucose, sialic acid and lactate.

4. Process according to one of the preceding claims, **characterized in that** said protoporphyrin is synthetic protoporphyrin IX.

5. Process according to one of the preceding claims, **characterized in that** said salts are chosen from potassium, magnesium, sodium, calcium, iron, zinc, cobalt and manganese salts.

6. Process according to the preceding claim, **characterized in that** said salts are chosen from: K₂HPO₄; KH₂PO₄; MgSO₄. 7 H₂O; Na₂HPO₄.12 H₂O; NaH₂PO₄. 2 H₂O; CaCl₂. 2 H₂O; FeSO₄. 7 H₂O; ZnSO₄. 7 H₂O; CoCl₂. 6 H₂O; MnSO₄. H₂O.

7. Process according to one of the preceding claims, **characterized in that** said amino acids are chosen from:
• arginine,
• alanine,
• at least one among: asparagine, glutamine, aspartic acid, glutamic acid,
• lysine,
• histidine,
• tryptophan,
• valine,
• isoleucine,
• leucine,
• tyrosine,
• phenylalanine,
• cystine or equivalent.

8. Process according to the preceding claim, **characterized in that** said culture medium comprises at least arginine, alanine, histidine, tryptophan, tyrosine, phenylalanine, cystine, aspartic acid and glutamic acid.

9. Process according to one of the preceding claims, **characterized in that** the cystine is replaced with glutathione or cysteine.

10. Process according to one of the preceding claims, **characterized in that** said vitamins are chosen from: thiamine, pantothenate, uracil, hypoxanthine, biotin, riboflavin and pyridoxine.

11. Process according to one of the preceding claims, **characterized in that** the arginine and the uracil are replaced with citrulline.

12. Process for preparing a vaccine composition, according to which:
- a) an antigen against *Haemophilus influenzae* type b (Hib), consisting of the capsular polysaccharide (PRP), is prepared, on an industrial scale, according to the process of Claim 1,
- b) the capsular polysaccharide obtained in step a) is conjugated to a carrier protein.

13. Process according to the preceding claim, **characterized in that** the carrier protein is tetanus toxoid.

14. Process according to either of Claims 12 and 13, **characterized in that** the conjugate obtained in step b) is combined with at least one or more antigens that are typically present in paediatric vaccines.

15. Process for preparing a vaccine composition according to Claim 12 or 13, **characterized in that** the conjugate obtained in step b) is combined with diphtheria, tetanus and hepatitis B antigens.

16. Process for preparing a vaccine composition according to Claim 12 or 13, **characterized in that** the conjugate obtained in step b) is combined with diphtheria toxoid, tetanus toxoid, and 2 acellular *Bordetella pertussis* antigens, which are toxoid and filamentous hemagglutinin.

17. Process for preparing a vaccine composition according to Claim 12 or 13, **characterized in that** the conjugate obtained in step b) is combined with diphtheria toxoid, tetanus toxoid, and 3 acellular *Bordetella pertussis* antigens, which are toxoid, pertactin and filamentous hemagglutinin.

18. Process for preparing a vaccine composition according to Claim 12 or 13, **characterized in that** the conjugate obtained in step b) is combined with diphtheria toxoid, tetanus toxoid, and 5 acellular *Bordetella pertussis* antigens, which are toxoid, pertactin, agglutinogens and filamentous hemagglutinin.

19. Process for preparing a vaccine composition according to Claim 12 or 13, **characterized in that** the conjugate obtained in step b) is combined with diphtheria toxoid, tetanus toxoid, 2, 3 or 5 acellular *Bordetella pertussis* antigens and also inactivated type 1, 2 and 3 polioviruses.

20. Process for preparing a vaccine composition according to Claim 12 or 13, **characterized in that** the conjugate obtained in step b) is combined with diphtheria toxoid, tetanus toxoid, hepatitis B antigens, 2, 3 or 5 acellular *Bordetella pertussis* antigens and also inactivated type 1, 2 and 3 polioviruses.

21. Process for preparing a vaccine composition according to Claim 12 or 13, **characterized in that** the conjugate obtained in step b) is combined with the whole *Bordetella pertussis* bacterium.

22. Process for preparing a vaccine composition according to Claim 12 or 13, **characterized in that** the conjugate obtained in step b) is combined with at least one of the antigens against diphtheria, tetanus, poliomyelitis, hepatitis B, infections caused by *Neisseria meningitidis* or by *Streptococcus pneumoniae.*
